# EUROPEAN PATENT SPECIFICATION

(11) **EP 1 782 065 B1**
(45) Date of publication and mention of the grant of the patent: **23.07.2008**
(21) Application number: 05776264.3
(22) Date of filing: 26.08.2005
(51) Int. Cl.: G01N 33/50, G01N 33/68

(54) **IDENTIFICATION OF COMPOUNDS FOR THE TREATMENT OF POSTERIOR CAPSULE OPACIFICATION**
IDENTIFIKATION VON VERBINDUNGEN ZUR BEHANDLUNG VON NACHSTAR
IDENTIFICATION DE COMPOSÉS POUR LE TRAITEMENT DE L'OPACIFICATION DE LA CAPSULE POSTERIEURE

(30) Priority: 27.08.2004 GB 0419145
(43) Date of publication of application: 09.05.2007
(73) Proprietor: The University Court of the University of Dundee, Dundee DD1 4HN (GB)
(72) Inventor: SPRUCE, Barbara, Ann, Rait, Perthshire PH2 7SB (GB); DUNCAN, George School of Biological Sciences, Norwich, Norfolk NR4 7TJ (GB); PRESCOTT, Alan, Kingskettle, Fife KY15 7PY (GB)
(74) Representative: Chapman, Paul Gilmour
(86) International application number: PCT/GB2005/003373
(87) International publication number: WO 2006/021811

(56) References cited:
- WO-A-20/04030653
- WO-A-20/04071185
- US-A1- 2004 052 823
- WONG T T L ET AL: "MMP inhibition prevents human lens epithelial cell migration and contraction of the lens capsule." THE BRITISH JOURNAL OF OPHTHALMOLOGY. JUL 2004, vol. 88, no. 7, July 2004 (2004-07), pages 868-872, XP002384038 ISSN: 0007-1161
- SPRUCE BARBARA A ET AL: "Small molecule antagonists of the sigma-1 receptor cause selective release of the death program in tumor and self-reliant cells and inhibit tumor growth in vitro and in vivo." CANCER RESEARCH. 15 JUL 2004, vol. 64, no. 14, 15 July 2004 (2004-07-15), pages 4875-4886, XP002384039 ISSN: 0008-5472 cited in the application
- QUINLAN M ET AL: "Phacoemulsification versus extracapsular cataract extraction: a comparative study of cell survival and growth on the human capsular bag in vitro." THE BRITISH JOURNAL OF OPHTHALMOLOGY. OCT 1997, vol. 81, no. 10, October 1997 (1997-10), pages 907-910, XP002384040 ISSN: 0007-1161
- WANG LIXIN ET AL: "Sigma receptor antagonists inhibit human lens cell growth and induce pigmentation." INVESTIGATIVE OPHTHALMOLOGY & VISUAL SCIENCE. APR 2005, vol. 46, no. 4, April 2005 (2005-04), pages 1403-1408, XP002384041 ISSN: 0146-0404
- MALOOF ANTHONY J ET AL: "Selective death of lens epithelial cells using demineralized water and Triton X-100 with PerfectCapsule sealed capsule irrigation: a histological study in rabbit eyes." ARCHIVES OF OPHTHALMOLOGY. OCT 2005, vol. 123, no. 10, October 2005 (2005-10), pages 1378-1384, XP009067331 ISSN: 0003-9950
- DUNCAN G ET AL: "Sigma receptor signalling in the human lens-role of the ER" IOVS, vol. 46, no. Suppl. S, 2005, page 4696, XP002384080 & ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404
- WANG L ET AL: "Role of sigma receptors in the human lens" IOVS, vol. 46, no. Suppl. S, 2005, page 2874, XP002384081 & ANNUAL MEETING OF THE ASSOCIATION-FOR-RESEARCH-IN-VISION-AND-OPH THALM OLOGY; FT LAUDERDALE, FL, USA; MAY 01 -05, 2005 ISSN: 0146-0404
- LIU C S ET AL: "A study of human lens cell growth in vitro. A model for posterior capsule opacification." INVESTIGATIVE OPHTHALMOLOGY & VISUAL SCIENCE. APR 1996, vol. 37, no. 5, April 1996 (1996-04), pages 906-914, XP002384042 ISSN: 0146-0404 cited in the application
- WORMSTONE I M ET AL: "Human lens epithelial cell proliferation in a protein-free medium." INVESTIGATIVE OPHTHALMOLOGY & VISUAL SCIENCE. FEB 1997, vol. 38, no. 2, February 1997 (1997-02), pages 396-404, XP002384043 ISSN: 0146-0404
- BOWEN W D: "Sigma receptors: recent advances and new clinical potentials" PHARMACEUTICA ACTA HELVETIAE, vol. 74, no. 2-3, March 2000 (2000-03), pages 211-218, XP002253858 ISSN: 0031-6865

## Description

### Field of the Invention

The invention relates to a method of testing agents, such as sigma receptor ligands for their potential use in treating posterior capsule opacification (PCO).

The present invention also relates to the use of sigma receptor ligands, such as sigma-1 receptor antagonists in the manufacture of a medicament for preventing posterior capsule opacification (PCO).

### Background to the Invention

Cataract extraction, followed by artificial lens implantation, is the commonest surgical procedure in the Western world. It is, however, beset by the major problem that in approximately 30-50% of cases the procedures need to be repeated, sometimes multiple times, due to a post-operative complication known as posterior capsule opacification (PCO) which is more common in younger patients (almost 100%). Additionally secondary intervention by Nd-YAG laser can itself result in further complications. PCO arises from the inappropriate growth of lens epithelial cells that normally line the anterior face of the "capsule" - the bag within which the lens lies. The capsule is left behind after cataract removal and is the receptacle for the artificial lens implant. PCO occurs when lens epithelial cells in the so-called "equatorial" region of the lens divide and migrate inappropriately along the posterior capsule; this induces wrinkling and progressive opacification of the posterior capsule with marked loss of visual acuity.

There is therefore a major clinical need to remove lens epithelial cells from the inside of the capsule, prior to artificial lens implantation, in order to prevent subsequent regrowth and migration of these cells. Laser therapy and physical "scraping" are used in an attempt to remove lens epithelial cells but these methods do not reliably remove 100% of lens epithelial cells and are also associated with complications such as retinal detachment. Another approach is to remove a segment of the posterior capsule prior to lens implantation; however, this has been associated with leakage of lens epithelial cells into the posterior chamber of the eye which could have detrimental consequences.

The use of medicaments - applied locally at the time of the operation - would in theory be another way to kill lens epithelial cells. However, such compounds would be required to be selectively toxic to the lens epithelial cells while sparing other ocular cell types and tissues that would be exposed due to local diffusion of the drug. It would also be highly advantageous for these compounds to kill lens cells by a specialized biological process termed apoptosis. Apoptosis causes cells to die in a way that avoids escape of noxious intracellular contents such as enzymes that could cause damage to the intraocular tissue. Unfortunately, however, the vast majority of agents that cause apoptosis are not selective for particular cell types and would be anticipated to cause the death of other cells, in addition to lens epithelial cells, within the eye. Additionally it has been shown that lens cells resident on the capsule are resistant to common apoptogens.

WO96/06863 disclosed that opioid receptor ligands induce apoptosis selectively in tumour cells and also in lens epithelial cells due to common properties of "self-reliance". In WO 96/06863 it was claimed that selective killing of lens epithelial cells by opioid receptor ligands would be medically useful in the perioperative management of cataracts to prevent or treat lens epithelial cell re-growth over lens implants, that is, the complication of posterior capsule opacification (PCO). Coating of the lens implant with opioids as a method of preventing or treating PCO was claimed as a particular embodiment.

WO00/00599 disclosed that sigma receptor ligands, which are distinct from opioid receptor ligands, kill selected cell populations including tumour cells and inflammatory cells and are therefore of use in the treatment of cancer and inflammatory disease.

WO01/74359 disclosed that sigma ligands modulate the survival of microvascular endothelial cells and that sigma-1 antagonists have an anti-angiogenic effect whereas sigma-1 agonists have a pro-angiogenic effect, both classes of agent therefore being of use in particular medical contexts.

WO02/079779 describes a screening method to identify compounds that would selectively kill tumour, microvascular endothelial and inflammatory cells, whilst sparing many normal cell types. This invention disclosed a cell-selective rise in calcium in lens epithelial cells. The death of lens epithelial cells (cultured in isolation from lens capsule) in response to sigma antagonists such as rimcazole has also been disclosed (Christopher Gribbon's PhD Thesis, University of Dundee 2002; and Spruce et al. Cancer Research 2004 Vol. 64 4875-4886).

Wong et al (2004) investigates whether inhibiting MMP activity can reduce lens epithelial cell migration and as a result, lead to a reduction in cell mediated capsule contraction. Ilomastat was found to significantly reduce lens epithelial cell migration onto the posterior capsule surface.

Nevertheless, the art has become more complex since these disclosures. It is now apparent that agents that kill primary cultures of lens epithelial cells by the process of apoptosis are not necessarily of use in vivo (in the clinical situation). This is because it has been discovered that the ocular lens capsule (the basement membrane on which lens epithelial cells reside) produces resistance to apoptosis induction by many apoptosis inducers (see for example Christopher Gribbon PhD Thesis University of Dundee 2002). This reveals that agents that are potent inducers of apoptosis when lens epithelial cells are cultured in isolation, fail to induce apoptosis in the same cells when these cells are in proximity to the anterior surface of an ocular lens capsule, in this case a bovine capsule. Importantly, it was discovered that apoptosis-inducing agents that are rescued by the capsule include sigma antagonists such as rimcazole. Rimcazole is substantially less able to induce apoptosis in lens epithelial cells in the presence of the bovine capsule. These findings therefore cast considerable doubt on the use of sigma receptor ligands as agents to induce apoptosis in lens epithelial cells and thereby prevent posterior capsule opacification. Thus, it remains a problem to find agents that will kill lens epithelial cells when in proximity to a lens capsule, whilst substantially not affecting/harming other cells types which are in close proximity.

It is amongst the objects of the present invention to obviate and/or mitigate at least one of the aforementioned disadvantages.

Without wishing to be bound by theory, the invention is based in part on reasoning by the present inventors that, since apoptosis inducers can be selectively rescued by different extracellular matrices (and basement membranes), a sigma-1 receptor antagonist, such as rimcazole would not necessarily be antagonised by the human ocular lens capsule. Furthermore, the inventors also postulated that primary cultures of lens epithelial cells that have been dissociated from and then reintroduced to the lens capsule might behave differently from lens epithelial cells that have remained in proximity with the lens capsule throughout.

There is provided use of a human capsular bag system for testing agents which selectively kill and/or inhibit human lens epithelial growth, wherein the human capsular bag system comprises a lens capsule within which lens epithelial cells remain, but from which the fibre cells of the lens have substantially been removed.

Removal of the capsular bag contents may be carried out, for example, by hydrodissection and phacoemulsification. Importantly, the above system comprises both the anterior and posterior faces of the lens capsule.

The inventors proved to be correct in their prediction that agents, such as sigma receptor antagonists would behave differently towards lens epithelial cells when present within the human capsular bag system, rather than when cultured in proximity to an anterior lens capsule from a non-human species. Rimcazole, for example, potently induces cell killing in human lens epithelial cells when contained in the human capsular bag system. Importantly, it was also discovered that a subset of lens epithelial cells are particularly susceptible: cells derived from the equatorial region which divide and migrate and are therefore particularly responsible for lens epithelial regrowth.

The capsular bag model system is particularly useful in testing sigma receptor ligands, such as sigma receptor antagonists or agonists for their ability to selectively inhibit lens epithelial growth or to induce lens cell killing

Preferably, the test agent/sigma receptor ligand is capable of inhibiting growth of the lens epithelial cells present in the lens capsule and more preferably capable of selectively killing the lens epithelial cells.

The invention provides an in vitro method of identifying an agent, such as a sigma receptor ligand, e.g. a sigma-1 receptor antagonist, potentially suitable for treating PCO, comprising the steps of:
a) using a so-called capsular bag system comprising a human lens capsule from which the lens contents - principally lens fibre cells - have substantially been removed but which still contains adherent lens epithelial cells;
b) contacting a test agent, typically a sigma receptor ligand, with said lens epithelial cells residing on said lens capsule; and optionally, lens epithelial cells cultured in the absence of a capsule; and
c) observing a reduction in viability or growth when said test agent, typically a sigma receptor ligand is contacted with said lens epithelial cells in proximity to the lens capsule.

Candidate sigma receptor ligands may first be identified by classical radioligand binding assays, such as disclosed in, for example, WO00/00599.

Typically, the test agents, such as sigma receptor ligands may also be contacted with other cells such as, corneal endothelial and trabecular meshwork or other normal human cells such as fibroblasts grown at low passage in order to ensure the selectively of the test agent/sigma receptor ligand, that is, that the test agent/sigma receptor ligand does not substantially kill and/or prevent growth of cells other than lens epithelial cells. Normal cells - with which lens epithelial cells are to be compared - are defined for the purposes of the invention as cells that have normal (typical) properties of survival and growth regulation and that therefore exclude lens epithelial cells and microvascular endothelial cells. Tumour cells and inflammatory cells are also excluded as control cells. A cell-selective effect of an agent would be determined when normal cells show an increase in viable cell number compared to baseline (pre-treatment) cell numbers over a period of time, preferably over 48-72 hours when exposed to the test agent. In contrast, lens epithelial cells would show substantially no change or a reduction in viable cell numbers when exposed to the test agent administered at the same concentration and with the cells under similar culture conditions, in particular at a similar cell density. Assays to reveal cell-selective growth inhibition or killing (cytotoxicity) are described, for example, in Spruce et al. Cancer Research 2004 and are understandable to those skilled in the art.

Typically the human lens capsule of the present invention may be supported on a solid substrate such as a plastics or glass surface, e.g. the surface of a petri dish. Sigma receptor ligands may be first identified as such by conducting, for example, a radioligand binding assay, such as described in WO 00/00599 to ascertain that the test agent is a sigma receptor ligand, before carrying out the method as described above. Importantly, sigma receptor radioligand binding assays determine only that an agent binds to sigma receptor sites and not whether it acts as an agonist or antagonist in functional terms (whether it acts to stimulate or inhibit the receptor). In WO 00/00599 it was disclosed that different sigma receptor subtypes - or even binding pockets on the same receptor molecule - can act in opposite ways to either promote or suppress apoptosis (cell death). Given that a number of sigma rececptor ligands bind to both sigma-1 and sigma-2 receptor sites (that act in opposition to regulate cell death), the balance of their functional activities at the two sites then becomes important. Without wishing to be bound by theory, the inventors propose that, when it comes to the identification of novel sigma receptor ligands of therapeutic usefulness for PCO, the functional endpoint is what matters (selective inhibition of growth or induction of death in lens epithelial cells). Thus, all-encompassing sigma receptor radioligand binding assays accompanied by functional growth inhibition /cell death assays in lens epithelial cells may lead to the identification of a therapeutically meaningful subset of sigma receptor ligands.

Contacting may simply be carried out by pipetting a solution comprising the test agent//sigma ligand onto the surface of the lens capsule and/or into the lens capsule itself, or washing or otherwise bathing the lens capsule in a solution comprising the test agent/sigma ligand.

Observing a reduction in growth or induction of cell killing by the said test agent on lens epithelial cells may be carried out for example using a microscope, such as a phase contrast microscope. This can be carried out using an automated system. Typically, the lens epithelial cells would be viewed before and after contacting with the test agent, in order to ascertain the effectiveness of the test agent at inhibiting growth the lens epithelial cells. Desirably the test agent not only inhibits lens epithelial cell growth, but also kills the lens epithelial cells.

Conveniently the test agent may also be tested in a cell viability assay, such as described in Spruce et al Cancer Research 2004 in order to ascertain whether or not the test agent also inhibits the growth and/or kills other cell types. Desirably the test agent should not substantially inhibit the growth and/or kill other "normal" cell types, such as corneal endothelial, trabecular meshwork cells or fibroblasts.

Optionally, the test agents may also be tested in a lens cell culture viability assay as disclosed, for example, in Spruce et al Cancer Research 2004) in which lens epithelial cells are grown in culture i.e. not within the lens capsule.

The present invention therefore provides a method for detecting test agents, such as sigma receptor ligands, which can selectively inhibit the growth and/or kill lens epithelial cells whilst substantially not inhibiting the growth and/or killing other normal cells, such as corneal endothelial cells, trabecular meshwork cells or fibroblasts.

It has been taught previously that so-called sigma-1 receptor antagonists have a particular value as agents to induce tumour cell death (Spruce et al Cancer Research 2004). Sigma-1 receptor antagonists are known as such in the art from a traditional pharmacological classification (based for example on inhibition of psychotic behaviour in animal models). Broadly speaking, the same class of agent (defined by traditional pharmacology) also causes selective antagonism of sigma-1 receptor-mediated repression of the death programme in tumour and microvascular endothelial cells. It is proposed herein that the same traditional pharmacological classification of sigma-1 receptor antagonists defines at least one subset of sigma receptor ligand that has therapeutic application in PCO.

Whereas sigma-1 receptor antagonists will broadly have activity against tumor and lens epithelial cells, it is clear that there are differences in potency amongst this class of agent that do not always correlate with affinity of interaction with the sigma receptor in standard radioligand binding assays. For example, the agent BD-1047 is a highly selective and potent sigma-1 receptor antagonist but is less potent than other sigma-1 receptor antagonists in its tumour cell killing properties. It has therefore been proposed that the subcellular localisation of the sigma receptor may contribute to the degree of susceptibility to sigma receptor ligands (Spruce et al Cancer Research 2004). Without wishing to be bound by theory, the present inventors postulated that differential subcellular localisation of the sigma receptor, or other factors, could lead to sigma receptor ligands being differentially potent in lens epithelial cells compared to tumour cells. As disclosed in more detail hereinafter, this prediction has turned out to be correct since the aforementioned compound BD-1047 (a highly selective sigma-1 receptor antagonist) is substantially more effective at killing lens epithelial cells compared to tumour cells. Specifically, the IC50 (the concentration of drug required to produce 50% growth inhibition) of BD-1047 for tumour cells is in the region 50-100 micromolar; in contrast, the IC50 for human lens epithelial cells (in the capsular bag system) is in the region of 10 micromolar. Thus, BD-1047 and/or other sigma-1 receptor antagonists may be agents to treat PCO even though they may be much less effective anti-tumour agents.

Experiments with rimcazole have revealed an additional mechanism by which the sigma-1 receptor antagonists act on lens cells. Concentrations of rimcazole that were sub- or semi-lethal caused an accumulation of melanin pigment granules in the lens cells. The melanin accumulation may be part of a differentiation programme that would also lead to an arrest of lens cells proliferation. Melanin accumulation could therefore act as a biomarker of response during or after a period of sigma antagonist treatment. The sigma-1 receptor antagonist BD1047 also induced pigmentation in the lens cells, indicating that the response is specific to sigma ligands.

Thus, in a further aspect, there is provided an in vitro method of testing and/or ensuring the efficacy of a sigma receptor ligand, typically a sigma-1 receptor antagonist, in treating/preventing PCO, comprising the steps of:
a) using a human capsular bag system comprising a lens capsule to which lens epithelial cells are adherent and from which the original lens contents (fibre cells) have substantially been removed;
b) contacting a sigma receptor ligand, typically a sigma-1 receptor antagonist with said lens epithelial cells within said lens capsule and optionally cultured lens epithelial cells without the capsule present; and
c) detecting any increase in pigmentation in the lens epithelial cells within said lens capsule and/or in cultured lens epithelial cells.

It is to be appreciated that the above method may be carried out visually, by comparing a degree of pigmentation of the lens epithelial cells, before and after contacting with the signa ligand. This may be a manual operation, or could, for example, be automated, using, for example, a CCD camera and appropriate software to compare changes in pigmentation between images. As mentioned above, any increase in pigmentation is postulated to be due to melanin production in the epithelial cells as a result of adding a sigma receptor ligand, such as sigma-1 receptor antagonist.

The present invention also provides use of at least one sigma-1 antagonist for the manufacture of a medicament for preventing and/or treating posterior capsule opacification (PCO).

Preferred sigma receptor ligands are Rimcazole and BD1047.

It is understood therefore that the sigma receptor ligand may be intended to be administered before, during and/or after cataract surgery. Generally speaking the sigma receptor ligand may be intended to be administered as a topical formulation. However, before and/or during surgery, the sigma receptor ligand could, for example, be injected, or otherwise administered within the lens capsule. Oral administration, as for example with rimcazole, is also a possibility.

Throughout the specification mention is made to sigma receptor ligands and sigma-1 receptor antagonists in general. Many compounds are known and/or can be identified as being sigma receptor ligands, by, for example, a sigma receptor ligand binding assay such as described in WO 00/00599. Preferred sigma receptor ligands include rimazole and related compounds as disclosed in US 4,379,160 and AU 201630, BD1063, BD1047, AC915, IPAG, NE-100, haloperidol, reduced haloperidol, BD-1008, BMY 14802, although this is not to be construed as limiting. The present invention also encompasses the use of salts and solvates of appropriate sigma receptor ligands and mixtures of sigma receptor ligands, such as a sigma-1 receptor antagonist and sigma-2 receptor agonist, providing such mixtures result in the selective inhibition of growth and/or killing of lens epithelial cells when in situ.

The present invention will now be further described by way of example and with reference to the figures which show:

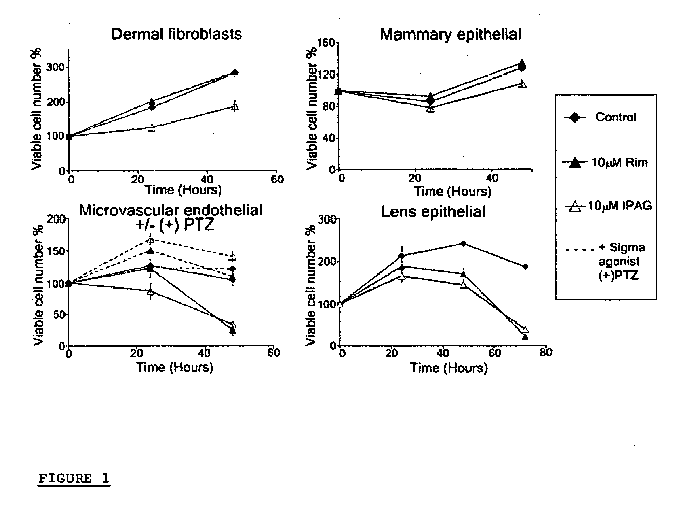

Figure 1 shows than lens epithelial cells are sensitive to sigma 1 receptor antagonists. Microvascular endothelial and lens epithelial cells resemble tumour cells in being susceptible to sigma-1 receptor antagonists. Human adult male dermal fibroblasts, adult mammary epithelial cells, adult dermal microvascular endothelial cells and bovine lens epithelial cells at low passage were exposed to 10 µM concentrations of the sigma-1 receptor antagonists rimcazole and IPAG for up to 72 hours. Change in cell viability over time was measured in the MTS assay; data points represent mean values (± SD), obtained from wells in triplicate, expressed relative to baseline (pre-treatment) values. Graphs depict representative experiments, performed at least three times. Microvascular endothelial cells were protected from rimcazole and IPAG by co-administration of equimolar concentrations of two prototypic sigma-1 receptor agonists (+)-pentazocine (PTZ, dotted lines) and (+)-SKF10,047 (not shown).

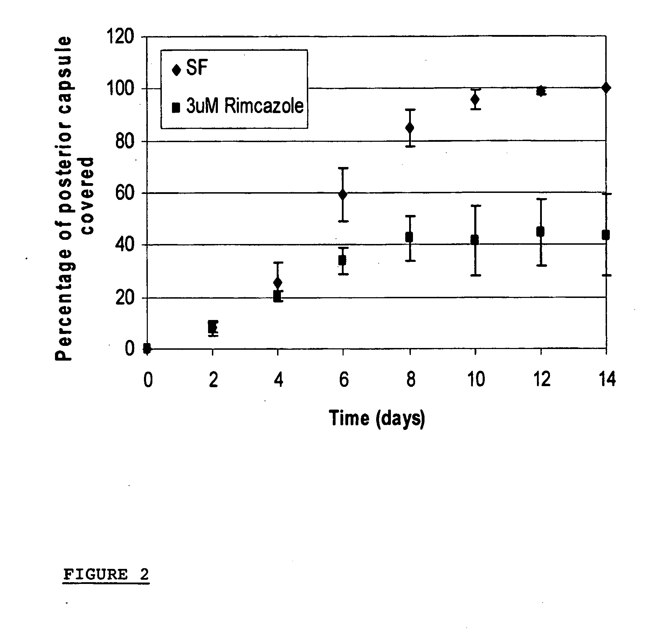

Figure 2 shows that low concentrations of the sigma 1 receptor antagonist Rimcazole inhibits growth of human lens epithelial cells on the posterior capsule. Rate of cell coverage of the posterior capsule beyond the rhexis; 100% represents confluency. Experiments were repeated on 4 occasions. The means and standard errors are displayed.

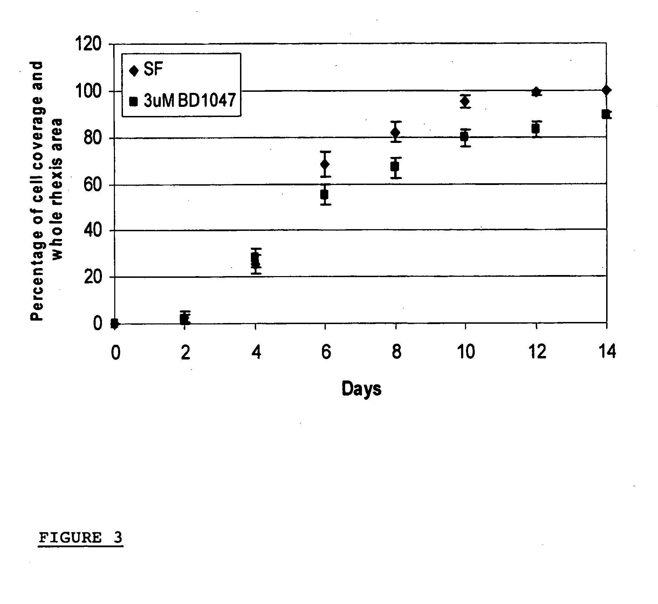

Figure 3 shows that low concentrations(equal to rimcazole concentration used in Fig. 2) of the sigma 1 receptor antagonist BD 1047 inhibit growth of human lens epithelial cells on the posterior capsule. Rate of cell coverage of the posterior capsule beyond the rhexis; 100% represents confluency. Experiments were repeated on 4 occasions. The means and standard errors are displayed.

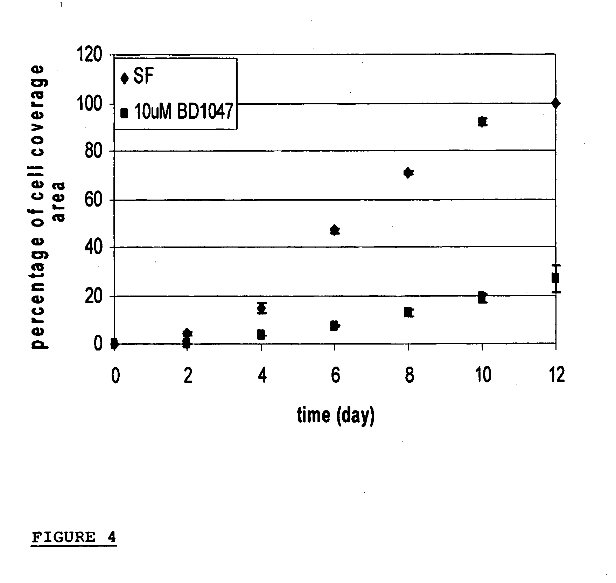

Figure 4 shows that lens epithelial cells have a profile of relative susceptibility to sigma antagonists that differs from tumour cells; 10 micromolar concentrations of the sigma 1 rececptor antagonist BD 1047 (substantially less than concentrations of BD-1047 required to inhibit human tumour cell growth) markedly inhibit growth of human lens epithelial cells on the posterior capsule. Rate of cell coverage of the posterior capsule beyond the rhexis; 100% represents confluency. Experiments were repeated on 4 occasions. The means and standard errors are displayed.

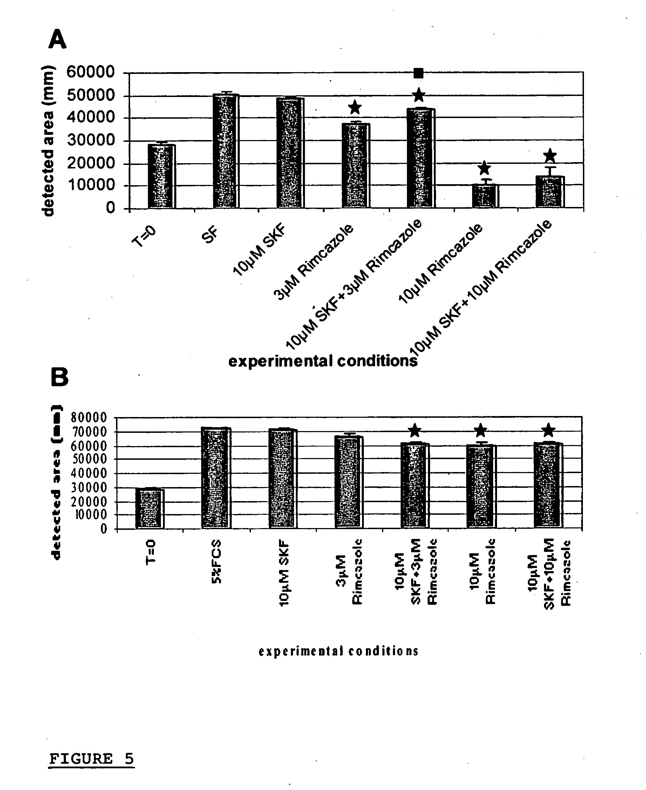

Figure 5 shows that the growth of the spontaneously immortal lens cell line FHL124 is inhibited by the sigma 1 receptor antagonist Rimcazole. The effect of sigma receptor ligands on human lens cell growth in FHL124 cell line maintained in serum-free media (A) or EMEM supplemented with 5%FCS (B). The sigma receptor agonist SKF10047 partially rescues the cells from Rimcazole inhibited growth at two concentrations. The cells were cultured for 4 days with experimental conditions. Experiments were repeated on 4 occasions. Data are expressed as Mean ± S.E.M., the star represents significant difference from untreated control with rimcazole, and box represents significant difference from untreated control with (±)SKF10047 (*t*test p<0.05). Note that 124 cells do not behave same as native.

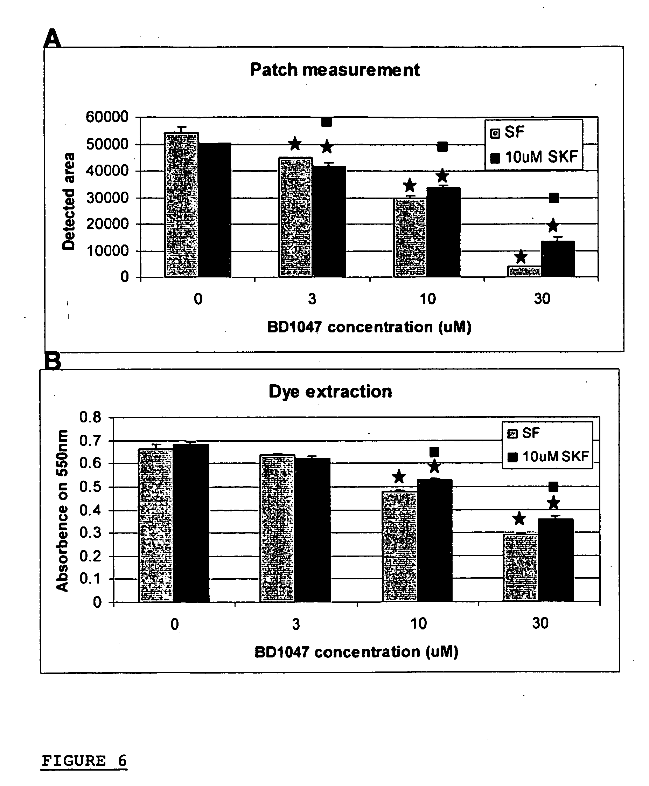

Figure 6 shows that the growth of the spontaneously immortal lens cell line FHL124 is inhibited by the sigma 1 receptor antagonist Rimcazole. Again SKF10047 partially inhibits the effect of Rimcazole. The effect of sigma-1 receptor ligands on human lens cell growth in FHL 124 cell line maintained in serum-free media. A represents the data of patch area measurement. B represents the dye extraction from each stained patch, ie. cell number. The cells were cultured for 4 days with experimental conditions. Experiments were repeated on 4 occasions. Data are expressed as Mean ± S.E.M, the star represents significant difference from untreated control with rimcazole, and ■ represents significant difference from untreated control with (±)SKF10047 (*t*test p<0.05).

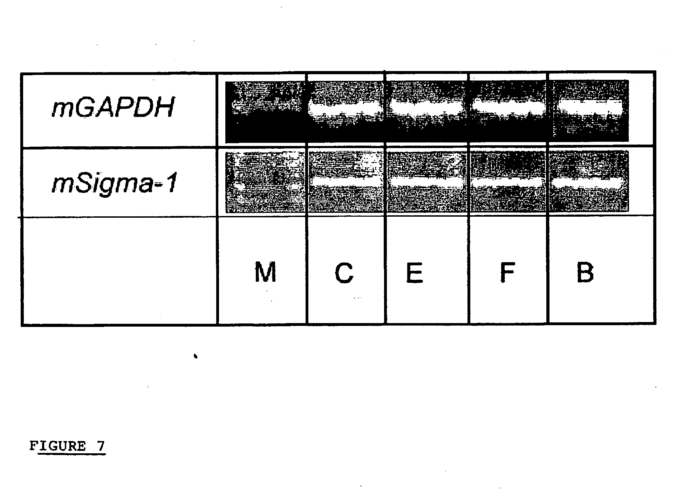

Figure 7 shows that the sigma 1 receptor is expressed in the intact lens and epithelial cells migrating across the posterior capsule. RT-PCR agarose gels showing the expression of sigma-1 receptor mRNA in the central anterior epithelium (C), equatorial epithelium (E), fibre cells (F) and ex vivo capsular bag (B); M represents markers.

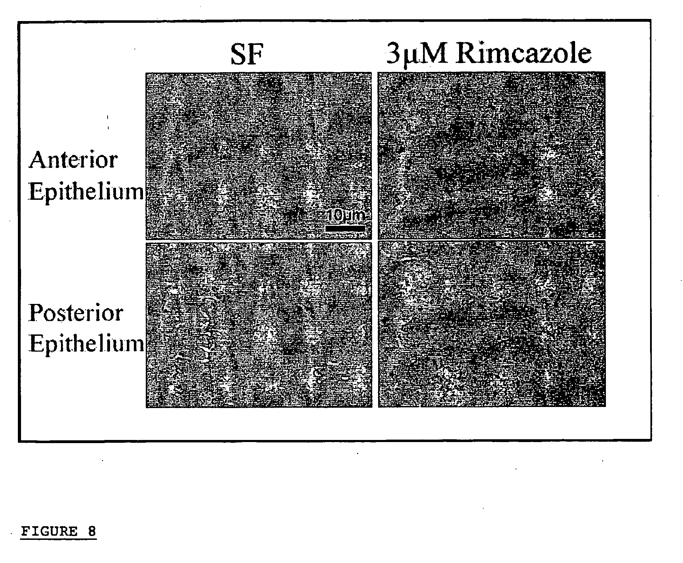

Figure 8 shows that epithelial cells on the posterior capsule become pigmented in response to a sub-lethal dose of the sigma-1 receptor antagonist Rimcazole. Phase micrographs of cultured human lens cells on central anterior (upper) and posterior capsule (lower) respectively. The cells were maintained in serum-free or supplemented (3µM Rimcazole) media for one week. The images were converted to grayscale with Adobe photoshop software.

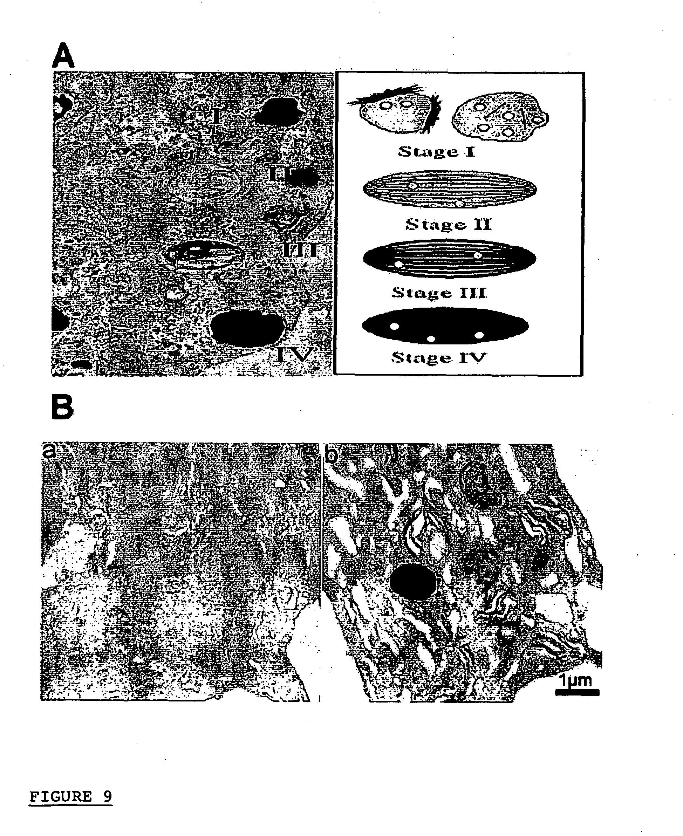

Figure 9 shows that TEM confirms that the Rimcazole treated cells contain pigment granules at various stages of maturation. Formation of pigment granules in human epithelial cells. A. Different stages of pigment granule formation (I-IV) as defined in MNT-1 melanoma cells by Seiji et al. 1963. B. Cross section of human lens epithelial cells grown in (a) control medium and (b) the presence of 3µM BD1047.

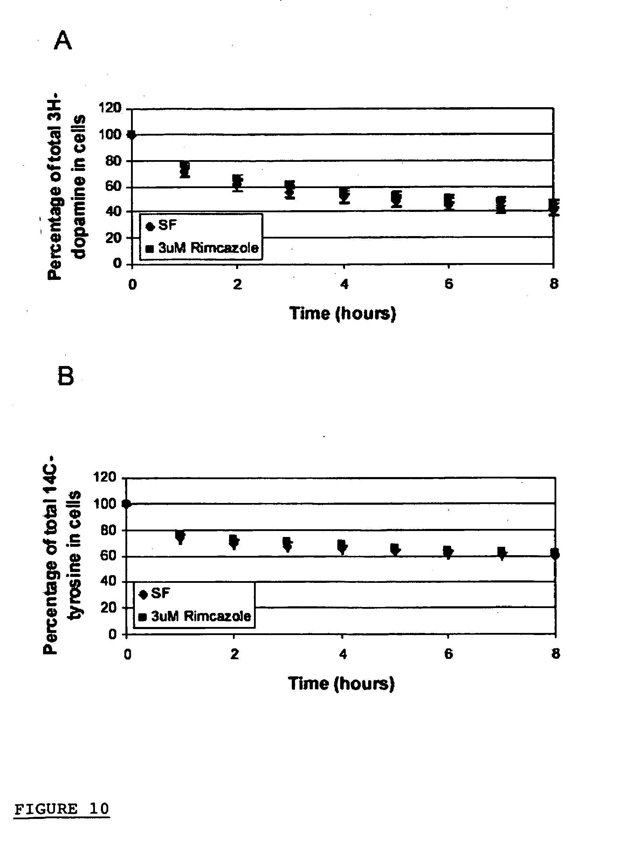

Figure 10 shows that Rimcazole has no effect on the efflux of Dopamine or Tyrosine from lens cells. Efflux of (A) ³H-dopamine and (B) ¹⁴C-tyrosine from human lens epithelial cells in control (SF) and rimcazole supplemented media. Data are presented as mean ± 6 separate experiments.

### Materials and Methods

### Anterior Lens Epithelium

The use of human tissue in the study was in accordance with the provisions of the Declaration of Helsinki. Human eye tissue donated for research was obtained from the East Anglian Eye Bank and the lens dissected from zonules and placed anterior side down onto a sterile 35-mm tissue culture dish. The area of the central anterior epithelium and underlying capsule was then carefully dissected out and transferred to a fresh 35mm culture dish where it was secured with pins.

### In vitro Capsular Bag Model

The model previously described by Liu et al (1996) was used. A sham cataract operation was performed on human donor eyes. The resultant capsular bag was then dissected free of the zonules and secured on a sterile 35mm PMMA petri dish. Eight entomological pins (D1: Watkins and Doncaster Ltd., Kent, UK) were inserted through the edge of the capsule to retain its circular shape. Incubation was at 35°C in a 5% CO₂ atmosphere. Ongoing observations were performed with a Nikon phase-contrast microscope and images captured with a digital camera (Coolpix 950; Nikon, Tokyo, Japan) with associated imaging software (Mr Y Zhu, personal communication). In some cases preparations were used for radioactive isotope studies.

### Growth Assay

Capsular bags were dissected and donor pairs checked for comparable cell coverage of the remaining anterior capsule by phase-contrast microscopy (see Liu et al for details). The bags were maintained in Eagle's minimum essential medium (EMEM) or EMEM supplemented with either 3µM Rimcazole dihydrochloride (rimcazole), 10µm(+)-SKF 10047 hydrochloride (SKF), and 3µM or 10 µM BD1047 dihydrochloride (BD1047) (all supplied by TOCRIS) and incubate at 35°C in a 5% CO₂ atmosphere. The medium was replaced every 2 days and ongoing observations and analyses were performed as above.

### Western Blot Analysis

After dissection, epithelial preparations were washed in serum-free (SF) EMEM then maintained in fresh EMEM and EMEM containing 3µM Rimcazole dihydrochloride (rimcazole) for 5 days. Cells were then lysed on ice in buffer: 50mM HEPES [pH 7.5], 150 mM NaCl, 1% Triton X-100, 1 mM EDTA, 10% glycerol, 10 mM sodium pyrophosphate, 2 mM sodium orthovanadate, 10 mM sodium fluoride, 1 mM phenylmethylsulfonyl fluoride (PMSF), and 10 µg/ml aprotinin. Lysates were pre-cleared by centrifuging at 1300rpm 4°C for 10 minutes, and the protein content of the soluble fraction was assayed by A Bicinchoninic acid protein assay (Pierce, Rockford, IL). Equal amounts of protein from each sample were loaded onto 10% SDA-PAGE gels for electrophoresis and transfer onto polyvinylidene difluoride (PVDF) membrane (NEN Life Science Products, Boston, MA) with a semidry transfer cell (Trans-Blot; Bio-Rad, Herts, UK). Proteins were detected using a chemiluminescent blot analysis system (ECL⁺ Amersham Biosciences, Amersham, UK) with Anti-tyrosinase (Upstate Biotechnology, Lake Placid, NY), Anti-tyrosinase related protein 1(TRP1) (SANTA CRUZ BIOTECHNOLOGY, INC), Anti-tyrosinase related protein 2 (TRP2) (SANTA CRUZ BIOTECHNOLOGY, INC) and Anti-Actin (SANTA CRUZ BIOTECHNOLOGY, INC).

### Reverse Transcription-Polymerase Chain Reaction

After dissection, epithelial preparations were washed in serum free EMEM, and RNA was collected from the cells by using a mini kit (RNeasy; Qiagen Ltd., Crawley, UK). RNA (250 ng) was reverse transcribed in a 20 µl reaction mixture (Superscript^{™} II RT; Invitrogen Ltd, Paisley, UK). cDNA (1µl; diluted 1 in 5 in sterile double distilled water) was amplified by PCT in a 20µl reaction buffer in the following condition: 0.5 µM each primer (Invitrogen Ltd, Paisley, UK), 0.8 mM deoxy-nucleoside trisphosphate mixture (Biolin Ltd, London, UK), 10 mM Tris-HCl, 1.5 mM MgCl₂, 50 mM KCl and 2.5 U TaqDNA polymerase (Roche Diagnostics, Lewes, UK). PCR was performed using the following program with a thermal controller (MJ Research Inc. Reno. NV): initial denaturation 95°C for 2 minutes; denaturation at 94°C for seconds; annealing at 55°C for 30 seconds; extension at 72°C for 40 seconds. Step 2 through 4 were cycled 27 times (GAPDH); 36 cycles (sigma 1 receptor) with a final extension at 72°C for 10 minutes. The oligonucleotide primer (5'-3') sequences specific for the genes examined were as follows:
GAPDH: ACCACAGTCCATGCCATCAC (sense) and TCCACCACCCTGTTGCTGTA (anti-sense); Sigma-1 receptor: 5'-AGCGCGAAGAGATAGC-3' (sense) and 5'-AGCATAGGAGCGAAGAGT-3' (anti-sense). PCT products, together with the 100bp DNA markers (Invitrogen-life Technologies), were run on a 1% agarose gel, and images were captured and analysed (1D system; Eastman Kodak, Rochester, NY).

### Measurement of Radioactive Isotopes

Capsular bags were maintained in Eagle's minimum essential medium (EMEM) or EMEM supplemented with 3µM Rimcazole dihydrochloride (rimcazole) (TOCRIS) for 7 days (when the pigment granules could be observed in cells). 1µCl/ml of ³H-dopamine and 2µCi/ml of ¹⁴C-tyrosine (Amersham Biosciences) were then added to each dish for an additional 24 hours. At the end point, 10µl of medium were collected from each dish, and transferred into scintillation vials. The bags were washed briefly twice with EMEM and fresh control or experimental media added for one hour. The medium was then collected into scintillation vials and the procedure repeated eights times. The experiment was then terminated by adding 1 ml of ice-cold 5% Trichloroacetic acid (TCA) (Fisher Chemicals) to each dish. After 30 minutes, the TCA was removed from each well to determine the cytosolic dopamine and tyrosine levels. 1 ml of 250mM NaOH was then added to each dish to determine the radio-isotope levels in the TCA precipitable fraction. 10 mls of scintillation fluid (Hisafe Supermix) were then added to each scintillation vial and the samples assayed using a Wallac scintillation counter with appropriate background controls. Results were expressed in disintegrations per minute (DPM).

### Experimental results

### In vitro cell death assays

The present inventors have tested a panel of sigma-1 receptor antagonists such as IPAG, BD1047, BD-1063 and BMY 14802 in addition to Rimcazole for their ability to kill lens cells grown on tissue culture plastic plates. These have been compared to the effect of these agents on other cell types, such as tumour cells, previously shown to be susceptible to sigma-1 antagonists as well as primary cells which are resistant to these drugs. Primary bovine lens cells in tissue culture are almost as susceptible to Rimcazole and IPAG as tumour cells (Figure 1).

### Capsular bag assays

Rimcazole inhibits growth of human lens cells on the posterior capsule in the present PCO model at doses comparable or less than the dose required to kill tumour cells (3µm: Figure 2). BD1047 at this dose is less inhibitory but at 10µm (still less than is needed to kill tumour cells) it potently inhibits growth of lens cells on the capsule (Figure 4).

### Cell growth assay

The growth of the spontaneously immortal human lens cell line FHL124 was investigated using a patch assay. Cells grown from a single patch derived from a coverslip and stained with dye to estimate the cell number. At both 3 and 10 µm Rimcazole growth is significantly inhibited in serum free medium but in 5% FCS growth inhibition is less marked at the higher dose (Figure 5). The specific sigma-1 agonist (+)-SKF1004partially rescues sigma receptor antagonist-mediated growth inhibition at both concentrations in serum free medium (Figure 5). (+)-SKF10047 also partially restores growth to BD1047 inhibited FHL124 cells at the higher concentrations (10 and 30 µm). (Figure 6). BD1047 is less effective at the lower dose (10µm) most of its effects seem to be on patch size rather than protein content (i.e. cell number, Figure 6). Attentuation of rimcazole and BD-1047-mediated growth inhibition by a highly specific sigma-1 receptor agonist confirms that growth inhibition is at least partly mediated through antagonism of sigma-1 receptor sites.

### Expression of Sigma-1 receptors in the lens

RT-PCR demonstrates sigma-1 receptor m-RNA in all regions of the human lens: both in the anterior and equatorial epithelium and in the fibre cells. Additionally epithelial cells migrating across the posterior capsule in the capsular bag model also express sigma-1 receptors (Figure 7).

### Effects of Sigma antagonists on pigmentation of lens cells

Observation of primary human lens epithelial cells on the posterior capsule during growth inhibition assays with the sigma receptor antagonists Rimcazole and BD1047 showed that these cells become pigmented while control cells remained clear. This pigmentation was also present in cells similarly tested on the rhexis (anterior capsule) removed during preparation of the capsular bag (Figure 8) and in the FHL-124 lens cell line (not shown). Transmission electron microscopy of these cells showed that the pigment was packaged into vesicles (Figure 10B) and appeared to follow the same developmental progression (stages 1-1V) as in MNT-1 melanoma cells (Figure 9A, Rapaso et al, 2002; Seiji et al. 1963) suggesting that the pigment was melanin. Sigma receptor antagonists have been reported to inhibit dopamine transport (Moison et al. 2003; Izenwasser, S. et al, 1993; Nuwayhid and Werling, 2003) but dopamine efflux was unaffected by Rimcazole in the capsular bag system (Figure 10A).

### Discussion

The results demonstrate that lens epithelial cells are very sensitive to sigma receptor antagonists and that this sensitivity is retained in cultured cells derived from the epithelium. Sigma receptor antagonists can induce cell death and inhibit cell growth both in culture and on the posterior capsule. Sigma receptor antagonists can thus limit cell coverage of the posterior capsule in the capsular bag model of PCO. The growth inhibitory effect of both rimcazole and the specific sigma-1 receptor antagonist BD-1047 is opposed by the sigma-1 receptor agonist (+)-SKF10,047. This confirms that the growth inhibitory effect of rimcazole and BD-1047 is mediated at least in part by antagonism at sigma-1 receptor sites. This is also supported by expression of the sigma-1 receptor in all regions of the intact lens including the fibre cells and its expression is retained by the epithelial cells on the posterior capsule (a position which they occupy only after cataract extraction).

Both the sigma-1 receptor specific antagonist BD-1 047 and Rimcazole induce pigmentation in the lens cells if administered at a sub-lethal dose. This pigmentation comprises melanin granules of the type seen in melanocytes and is likely to be the result of the upregulation of two enzymes in the melanin synthetic pathway, Tyrosinase and TYRP1, while TRP2 is unaffected, (data not shown). Thus lens epithelial cells have the ability to make pigment which may be suppressed by agonistic signalling at the sigma-1 receptor.

The sigma receptor antagonists used in this study arc potential candidates for the treatment of PCO and as lead compounds for the development of effective strategies for this common problem.

Melanin accumulation may be used as a marker of transdifferentiation of lens epithelial cells; thus, melanin induction could be a marker of cell cycle exit - that is to say, melanin induction is consistent with proliferation inhibition which, along with apoptosis induction would be a desirable outcome in the context of therapy for PCO.

Together these data suggest therefore that a subset of agents that may be particularly useful for treatment of PCO would be sigma receptor ligands that, when administered at sublethal concentrations induce melanin (a marker of proliferation inhibition) within lens epithelial cells. Such agents may be identified by a combination of standard radioligand binding assays to identify sigma receptor binding activity (as described in WO 00/00599) and the induction of melanin within cultured lens epithelial cells (using the techniques described herein).

Advantageously the determination of melanin accumulation in residual lens epithelial cells may be used as biomarker of response to rimcazole and/or other test agents of the invention. Such an assessment would be useful as an efficacy surrogate for clinical trials in PCO.

### References

Gribbon, C PhD Thesis University of Dundee 2002.
Liu CS, Wormstone IM, Duncan G, Marcantonio JM, Webb SF, Davies PD. A study of human lens cell growth in vitro. A model for posterior capsule opacification. Invest Ophthalmol Vis Sci. 1996 Apr;37(5):906-14.
M. Seiji, S. Iwashita, J Biochem (Tokyo) 54, 465 (Nov, 1963).
Rapaso, G. et al. 2002 Cell Struct. Funct. Vol. 27 pp443-456.
Moison et al. 2003; Neuropharmacol. Vo. 45 pp945-953.
Izenwasser, S. et al 1993 Eur. J. Pharmacol. Vol. 243 pp201-205.
Nuwayhid, S.J.and Werling, L.L. 2003 J. Pharmacol. Exp. Ther. Vol. 306 pp934-940.
Spruce et al 2004 Cancer Research Vol. 64 4875-4886
WO96/06863
WO00/00599
WO01/74359
WO02/079779
Wong, T.T.L. et al; The British Journal of Ophthalmology, 2004, Vol. 88, No. 7, p868-872.

## Claims

1. An in vitro method of identifying a sigma receptor ligand, potentially suitable for treating PCO, comprising the steps of:
a) using a so-called capsular bag system comprising a human lens capsule from which the lens contents - principally lens fibre cells - have substantially been removed but which still contains adherent lens epithelial cells;
b) contacting a sigma receptor ligand, with said lens epithelial cells residing on said lens capsule; and optionally, lens epithelial cells cultured in the absence of a capsule; and
c) observing a reduction in viability or growth when said sigma receptor ligand, is contacted with said lens epithelial cells in proximity to the lens capsule.

2. The method according to claim 1 wherein the sigma receptor ligand is a sigma-1 antagonist.

3. The method according to claims 1 or 2 wherein the sigma ligands are also contacted with other cells, such as corneal endothelial and trabecular mesh work or other normal human cells in order to ensure the selectivity of the sigma ligand.

4. The method according to any preceding claim wherein the lens capsule is supported on a solid substrate.

5. The method according to any preceding claim wherein contacting is carried out by pipetting a solution comprising the test agent onto the surface of the lens capsule and/or into the lens capsule itself, or washing or otherwise bathing the lens capsule in a solution comprising the test agent.

6. The method according to any preceding claim wherein observing a reduction in growth or induction of cell killing by the said test agent on the lens epithelial cells, is carried out using a microscope.

7. The method according to any preceding claim wherein the test agent is also tested in a lens cell culture viability assay.

8. An in vitro method of testing and/or ensuring the efficacy of a sigma receptor ligand, in treating/preventing PCO, comprising the steps of:
a) using a human capsular bag system, comprising a lens capsule to which lens epithelial cells are adherent and from which the original lens contents (fibre cells) have substantially been removed;
b) contacting a sigma receptor ligand, with said lens epithelial cells within said lens capsule and optionally cultured lens epithelial cells without the capsule present; and
c) detecting any increase in pigmentation in the lens epithelial cells within said lens capsule and/or in cultured lens epithelial cells.

9. The method according to claim 8 wherein the sigma receptor ligand is a sigma-1 receptor antagonist.

10. The method according to claims 8 or 9 wherein the detection of any increase in pigmentation in the lens epithelial cells is carried out by an automated system, using, for example, a CCD camera and appropriate software to compare changes in pigmentation between images.

11. Use of at least one sigma-1 antagonist, for the manufacture of a medicament for preventing and/or treating posterior capsule opacification (PCO).

12. The use according to claim 11 wherein the sigma receptor ligand is rimcazole, or BD1047 ([2-(3,4-dichlorophenyl)ethyl]-N-methyl-2-(diamino)ethylamine).

## Patentansprüche

1. In vitro-Methode zum Identifizieren eines Sigmarezeptorliganden, der potentiell für die Behandlung von Nachstar (PCO) geeignet ist, umfassend die Schritte des:
a) Verwendens eines so genannten Kapselbeutelsystems umfassend eine menschliche Linsenkapsel, aus der die Linseninhalte - hauptsächlich die Linsenfaserzellen - im Wesentlichen entfernt worden sind, die jedoch immer noch anhaftende Linsenepithelzellen enthält;
b) Kontaktieren eines Sigmarezeptorliganden mit den Linsenepithelzellen, die sich auf der Linsenkapsel befinden; und wahlweise mit Linsenepithelzellen, die in Abwesenheit einer Kapsel gezüchtet worden sind; und
c) Beobachten einer Reduktion der Lebensfähigkeit oder des Wachstums, wenn der Sigmarezeptorligand mit den Linsenepithelzellen in der Nähe der Linsenkapsel kontaktiert wird.

2. Methode nach Anspruch 1, wobei der Sigmarezeptorligand ein Signia-1-Antagonist ist.

3. Methode nach den Ansprüchen 1 oder 2, wobei die Sigmaliganden auch mit anderen Zellen, wie beispielsweise Hornhautetrdothelzellen und iridokornealem Balkenwerk oder anderen normalen menschlichen Zellen in Kontakt gebracht werden, um die Selektivität des Sigmaliganden sicherzustellen.

4. Methode nach einem der vorhergehenden Ansprüche, wobei die Linsenkapsel auf einem festen Substrat getragen wird.

5. Methode nach einem der vorhergehenden Ansprüche, wobei das Kontaktieren durch Pipettieren einer Lösung, die das Testagens umfasst, auf die Oberfläche der Linsenkapsel und/oder in die Linsenkapsel selbst oder Waschen oder auf andere Weise Baden der Linsenkapsel in einer das Testagens umfassenden Lösung durchgeführt wird.

6. Methode nach einem der vorhergehenden Ansprüche, wobei das Beobachten einer Reduktion des Wachstums oder der Induktion von Zellabtötung durch das Testagens auf den Linsenepithelzellen unter Anwendung eines Mikroskops durchgeführt wird.

7. Methode nach einem der vorhergehenden Ansprüche, wobei das Testagens auch in einem Linsenzellkulturlebensfähigkeitsassay getestet wird.

8. In vitro-Methode zum Testen und/oder Sicherstellen der Wirksamkeit eines Sigmarezeptorliganden beim Behandeln/Verhindern des PCO umfassend die Schritte des:
a) Anwendens eines menschlichen Kapselbeutelsystems umfassend eine Linsenkapsel, an der Linsenepithelzellen anhaften und von der die ursprünglichen Linseninhalte (Faserzellen) im Wesentlichen entfernt worden sind;
b) Kontaktierens eines Sigmarezeptorliganden mit den Linsenepithelzellen innerhalb der Linsenkapsel und wahlweise gezüchteten Linsenepithelzellen ohne Vorliegen der Kapsel; und
c) Erfassens irgend einer Erhöhung der Pigmentierung der Linsenepithelzellen innerhalb der Linsenkapsel und/oder der gezüchteten Epithelzellen.

9. Methode nach Anspruch 8, wobei der Sigmarezeptorligand ein Sigma-1-Rezeptorantagonist ist.

10. Methode nach den Ansprüchen 8 oder 9, wobei die Erfassung irgend einer Erhöhung der Pigmentierung der Linsenepithelzellen durch ein automatisiertes System unter Anwendung beispielsweise einer CCD-Kamera und geeigneter Software zum Vergleichen von Änderungen der Pigmentierung zwischen Bildern durchgeführt wird.

11. Verwendung von mindestens einem Sigma-1-Antagonisten für die Herstellung eines Medikaments zur Verhinderung und/oder Behandlung von Nachstar (PCO).

12. Verwendung nach Anspruch 11, wobei der Sigmarezeptorligand Rimcazol oder BD1047([2-(3,4-Dichlarphenyl)ethyl]-N-methyl-2-(diamino)ethylamin) ist.

## Revendications

1. Procédé *in vitro* pour l'identification d'un ligand du récepteur sigma potentiellement approprié au traitement de l'OCP, qui comprend les étapes consistant à:
a) utiliser un dit système à sac capsulaire comprenant une capsule du cristallin humain de laquelle le contenu - principalement les cellules fibreuses du cristallin - a été en grande partie enlevé mais qui contient encore des cellules épithéliales adhérentes du cristallin;
b) mettre en contact un ligand du récepteur sigma avec lesdites cellules épithéliales du cristallin résidant sur ladite capsule du cristallin et, en option, mettre en culture les cellules épithéliales du cristallin en l'absence d'une capsule: et
c) observer une réduction de la viabilité ou de la croissance quand ledit ligand du récepteur sigma est mis en contact avec lesdites cellules épithéliales du cristallin à proximité de la capsule du cristallin.

2. Procédé selon la revendication 1, où ledit ligand du récepteur sigma est un antagoniste du récepteur sigma-1.

3. Procédé selon les revendications 1 ou 2, où les ligands du récepteur sigma sont également mis en contact avec d'autres cellules, comme des cellules endothéliales de la cornée et le réseau trabéculaire ou avec d'autres cellules humaines normales pour assurer la sélectivité du ligand du récepteur sigma.

4. Procédé selon l'une quelconque des revendications précédentes, où la capsule du cristallin est supportée sur un substrat solide.

5. Procédé selon l'une quelconque des revendications précédentes, où la mise en contact est effectuée en pipetant une solution qui comprend l'agent à l'essai sur la surface de la capsule du cristallin et/ou dans la capsule du cristallin elle-même, ou en lavant ou en baignant autrement la capsule du cristallin dans une solution qui comprend l'agent à l'essai.

6. Procédé selon l'une quelconque des revendications précédentes, où l'observation d'une réduction de la croissance ou de l'induction d'une mort cellulaire par ledit agent à l'essai sur les cellules épithéliales du cristallin est effectuée en utilisant un microscope.

7. Procédé selon l'une quelconque des revendications précédentes, où l'agent à l'essai est également testé par une analyse de la viabilité de cellules du cristallin en culture.

8. Procédé *in vitro* permettant de tester et/ou de s'assurer de l'efficacité d'un ligand du récepteur sigma dans le traitement/la prévention de l'OCP, qui comprend les étapes consistant à:
a) utiliser un système à sac capsulaire humain qui comprend une capsule de cristallin à laquelle des cellules épithéliales du cristallin sont adhérentes et de laquelle le contenu originel du cristallin (cellules fibreuses) a été en grande partie enlevé;
b) mettre en contact un ligand du récepteur sigma avec lesdites cellules épithéliales dans ladite capsule du cristallin et, en option, avec des cellules épithéliales du cristallin cultivées sans qu'une capsule ne soit présente; et
c) détecter toute augmentation de la pigmentation des cellules épithéliales dans ladite capsule du cristallin et/ou dans les cellules épithéliales du cristallin en culture,

9. Procédé selon la revendication 8, où ledit ligand du récepteur sigma est un antagoniste du récepteur sigma-1.

10. Procédé selon les revendications 8 ou 9, où la détection de toute augmentation de la pigmentation des cellules épithéliales du cristallin est effectuée par un système automatisé qui utilise par exemple une caméra CCD et un logiciel approprié pour comparer les changements de pigmentation d'une image une autre.

11. Utilisation d'au moins un antagoniste du récepteur sigina-1 dans la fabrication d'un médicament conçu pour prévenir et/ou traiter une opacification de la capsule postérieure (OCP).

12. Utilisation selon la revendication 11, où le ligand du récepteur sigma est le rimcazole ou BD1047 ([2-(3,3-dichlaraphényl)éthyl]-N-méthyl-2-(diamino)éthylamine).
